Europäisches Patentamt

European Patent Office

Office européen des brevets

(11) Publication number: **0 134 798**
**B1**

(12) **EUROPEAN PATENT SPECIFICATION**

(45) Date of publication of patent specification: **08.06.88**

(21) Application number: **84900595.4**

(22) Date of filing: **29.12.83**

(86) International application number:
**PCT/US83/02071**

(87) International publication number:
**WO 84/02923 02.08.84 Gazette 84/18**

(51) Int. Cl.⁴: **C 12 Q 1/04, C 12 Q 1/14, C 12 Q 1/22, G 01 N 33/04, A 23 C 3/08, A 23 L 3/34**

(54) **A preservative composition for addition to a milk sample for compositional testing, and a method of screening milk samples employing it.**

(30) Priority: **28.01.83 US 461721**

(43) Date of publication of application:
**27.03.85 Bulletin 85/13**

(45) Publication of the grant of the patent:
**08.06.88 Bulletin 88/23**

(84) Designated Contracting States:
**DE FR GB SE**

(56) References cited:
DE-A-1 947 951
FR-A-1 084 995
GB-A- 8 551
GB-A- 360 360
GB-A-1 057 131

MILCHWISSENSCHAFT, vol. 34, no. 1, 1979, pages 14-16; Y. ARDO: "Bronopol as a preservative in milk samples"

MILCHWISSENSCHAFT, vol. 36, no. 2, 1981, pages 65-68; J. THOMASOW et al.: "Bestimmung des Fett-, Eiweiss- und Milchzuckergehaltes von Milch durch Messung der Infrarotabsorption mit dem Gerät Multispec"

(73) Proprietor: **DAIRY AND FOOD LABS, INC.**
**1750 Folsom Street**
**San Francisco, CA 94103 (US)**

(72) Inventor: **YOUNG, Randall, S.**
**2 Dead Horse Canyon Road**
**Lafayette, CA 94549 (US)**
Inventor: **FREDERICKS, Ronald, D., Jr.**
**1147 Davis Street**
**9 Redwood City, CA 94601 (US)**

(74) Representative: **Dipl.-Phys.Dr. Manitz Dipl.-Ing., Dipl.-Wirtsch. Finsterwald Dipl.-Chem.Dr. Heyn Dipl.-Phys. Rotermund Morgan, B.Sc.(Phys.) Robert-Koch-Strasse 1**
**D-8000 München 22 (DE)**

(56) References cited:
N, Hydrogen Ions Their Determination and Importance in pure and Industrial Chemistry, issued 1956, Hubert T.S. Britton, pages 340-348.

Courier Press, Leamington Spa, England.

## 0 134 798

**Description**

The present invention relates generally to a preservative composition for addition to a milk sample while it is fresh prior to sending it to a laboratory for compositional testing, the preservative composition comprising a compound for inhibiting bacterial degradation of the milk. A preservative of this kind is described in Milchwissenschaft 36(2) 1981 at pages 65—68. The present invention also relates to a method for screening milk samples in a compositional testing laboratory for bacterial degradation prior to testing.

It is common practice in the dairy industry to send samples of various dairy products to centralised laboratories for compositional testing. Typical measurements include the content of fat, protein, lactose, total solids, and added water. The samples may be further subjected to direct microscopic somatic cell count (DMSCC) which measures the content of bovine epithelial cells and serves as an indication of udder infection.

The test samples can be in transit for one or more days between the dairy farm and the central testing laboratory, and, even when refrigerated, are subject to various forms of bacterial degradation.

The most common form of bacterial degradation encountered arises from the growth of lactic acid bacteria, in particular streptococci and lactobacilli. These bacteria are present in raw milk and utilise lactose to produce lactic acid. Their ability to produce acid and proliferate at low pH favors their survival over other common organisms in milk and other dairy products. As the pH of milk is lowered, casein, the principal milk protein having an isoelectric point at a pH of 4.6, begins to precipitate. The precipitation is first noticeable as a very fine graininess at a pH of about 5.2 (fresh milk has a pH of 6.7). The milk forms a solid gel at a pH of about 4.6—4.7.

Another form of bacterial degradation is caused by proteolytic microorganisms which enzymatically denature milk protein. Proteolysis is accompanied by acid production when caused by certain species of *Micrococcus* and (to a lesser extent) by species of *Bacillus* and other groups. Such proteolytic degradation will predominate under conditions which are unfavorable to the growth of lactic acid bacteria.

The bacterial degradation of a dairy sample prior to testing is undesirable for two primary reasons. First, such degradation can directly affect the composition of the sample. For example, lactic acid conversion can reduce the lactose measurement while protein precipitation can lower the measured protein content. Extensive growth of proteolytic and lipolytic bacteria can also lower protein and fat measurements by denaturing these components.

Second, and more importantly, the presence of even small amounts of precipitated protein can foul the test equipment. Such test equipment is typically automated and relies on the transfer of fluid milk samples in narrow diameter capillary tubing. Precipitated protein will quickly clog such tubing, requiring that the test equipment be shut down while the passageways are cleared. Moreover, since the test equipment usually measures light absorption through the sample, precipitated protein will increase the observed light absorption leading to a false reading.

To overcome the problems associated with bacterial degradation of the milk samples, preservative (anti-bacterial) compositions are usually added to the samples at the dairy. A number of such preservatives are commonly in use, including potassium dichromate, boric acid, and 2-bromo-2-nitropropane-1,3-diol. While generally effective, individual milk samples can still suffer from bacterial degradation, particularly if they have been held for extended periods without refrigeration. Under such circumstances, it is very easy for the central testing laboratory to allow degraded samples into their test instrumentation. It is very difficult to detect the early stages of degradation when only a small amount of the protein has precipitated. Such incipient precipitation, however, can adversely effect the measurements taken and foul the test instrumentation as described above.

For the above reasons, it is the principal object of the present invention to provide a screening technique which will allow the personnel at the central test facility to easily determine when individual milk samples have been degraded and are unsuitable for testing.

In order to satisfy this object there is provided a preservative of the initially named kind which is characterised in that an indicator compound or a combination of indicator compounds compatible with said degradation inhibiting compound is incorporated in the preservative composition for sample screening prior to compositional testing, wherein said indicator compound, when added to the milk changes color over the pH range from 6.6 to 4.6, whereby bacterial degradation results in an observable change of color in the milk.

Also according to the present invention there is provided a method for screening milk samples in a laboratory for bacterial degradation prior to compositional testing, said method employing a preservative composition comprising a degradation inhibiting compound and an indicator compound or a combination of indicator compounds compatible therewith which changes color in aqueous solution over the pH range from 4.6 to 6.6, said method comprising: adding a preselected amount of the preservative composition to the milk samples while said samples are substantially fresh; observing the color of each milk sample immediately prior to compositional testing; and comparing the observed color to color standards which are indicative of the degree of bacterial degradation.

The present invention is thus based, amongst other things on the recognition that even samples of milk treated with preservative compositions to prevent bacterial contamination can be subject to bacterial contamination. Furthermore, the present invention recognises that such samples become unsuitable for

2

testing when bacteria consume the constituents to be analysed, such as lactose and protein, and when they act on the milk protein to change its degree of solubility. The majority of microorganisms capable of decomposing milk exhibit some level of acid production, and the present invention relies on the detection of incipient acid production as an indication of when a particular milk sample has undergone unacceptable degradation. The color indicator is combined with a preservative composition for simultaneous addition to the milk sample while it is still fresh, usually at the dairy.

The presently proposed preservative composition is useful with all liquid (non-cultured) milk products which would be expected to be free of lactic acid. Such products are typified by raw whole milk; homogenised milk, including whole milk, partly-skimmed milk, and skimmed milk; cream and the like. The presently proposed preservative composition will not be useful on those dairy products, such as cheese, cottage cheese, and yogurt, which have been cultured and in which lactic acid would naturally be present.

The presently proposed preservative composition thus allows detection of bacterial degradation by any species capable of acid production in milk and milk products. As stated above, the most common organisms are streptococci and lactobacilli, which convert lactose to lactic acid. Certain species of *Bacillus* responsible for sweet-curd formation, however, also produce lactic acid in sufficient amounts to allow detection by the proposed method. Other common microorganisms responsible for the degradation of milk and which produce detectable amounts of acid include certain strains of micrococci, microbacteria, pseudomonas and enteric bacteria. Some of these strains are also protolytic and/or lipolytic.

The color indicator, i.e. the indicator compound, selected for the presently proposed preservative will be sensitive to small amounts of acid production in milk to allow the early detection of potentially degrading organisms. The color indicator should display a perceptible color change over the pH range between fresh milk and milk which is about to undergo protein precipitation. Moreover, the color indicator should function with all types of acid-producing degradation, in particular those caused by lactic bacteria and sweet-curd forming bacteria. While the particular color response to each of these spoilage mechanisms may differ, the color response should be repeatable for each of the mechanisms. In this way, an observed color transition allows the person testing the sample to visually assess suitability of the sample for testing. Samples having unacceptable bacterial levels which result in degradation of the sample can thus be identified and separated prior to testing.

Suitable color indicators will display color transition over the pH range from 6.7 (the pH of fresh milk) to 4.6 (the isoelectric point of casein). Color transition should be most apparent in the pH range from about 6.1 to 5.1 where protein precipitation is most likely to commence. Protein precipitation as a result of lactic acid formation by lactic bacteria usually begins at a pH in the range from about 5.1 to 5.2, while precipitation resulting from sweet-curd bacteria is more variable and can occur at a pH as high as about 5.9 to 6.7.

Conveniently, the color indicator will provide a strong coloration of the fresh milk product as an indication that the preservative composition has been added. In this way, accidental consumption of the preserved milk samples will be avoided.

Fortunately, suitable indicator compounds are available in the art in the form of compounds which have previously been used for testing milk samples per se, i.e. analysis thereof. In this connection reference should be made to GB—A—360,360, FR—A—1 084 995 and pages 340—348 of the book "Hydrogen Ions—Their Determination and Importance in Pure and Industrial Chemistry, issued 1956 by Hubert T. S. Britton, GB—A—360 360 discloses that colorimetric methods have been proposed to diagnose udder ailments, in which the milk from the different teats is treated with an indicator by means of which in the case of any trouble other colours than the normal one, are produced (page 1, lines 56—62) and proposes a test liquid comprising an indicator the transmission of which lies between pH=6 and pH=8 (page 1, line 90 to page 2, line 32), bromothymol blue being mentioned as a particularly suitable indicator for these purposes.

FR—A—1 084 995 reveals a reagent permitting indication of the lactic acid level of milk by colorimetry comprising bromothymol blue (a) and bromocresol purple (c) (page 1, left column) and acceptance of normal milk only within a few seconds (page 1, last paragraph, page 2, first paragraph).

The book by Hubert T. S. Britton describes the use by Baker and Van Slyke of bromocresol purple for the examination of milk. When 1 drop of a saturated aqueous solution is added to 3 cm³ of milk, normal fresh milk assumes a greyish-blue colour. More alkaline milks, such as those drawn from diseased udders, or those which have been watered or skimmed, or those to which salts having an alkaline reaction have been added, produce a darker colour and should consequently arouse suspicion. More acid milks, due either to developed acidity, or to having been heated above the usual temperature of pasteurisation, give rise to lighter colours. Baker and Van Slyke also studied the rate of development of acid due to the bacteria contained in the milk by following the colour changes of the indicator after incubation in sterilised vessels for different periods of time at a given temperature. . . Similar observations have been made by Cooledge (Michigan Agr. Exp. Station, Techn. Bull., 52, 1921) using bromo-thymol blue. He has also devised a test of the keeping quality by incubating at 37°C. 0.1 cm³ of milk in a tube of broth containing this indicator and making colorimetric pH measurements every hour for eight hours.

Indicator compounds specifically suitable for the present proposal include bromcresol purple, chlorophenol red, nitrazine yellow and bromothymol blue which are available from J. T. Baker Chemical Co., Phillipsburg, N. J., as product nos. C950, F334, R778 and D470, respectively. Combinations of the color

**0 134 798**

indicators are also suitable and can provide enhanced color transition over a desired segment of the pH range.

As mentioned the degradation inhibiting compound used in the present invention must be compatible with the indicator compound. In particular, the degradation inhibiting compound should not interfere with the coloration or the color change brought about by the indicator compound. Suitable degradation inhibiting compounds, which display little or no coloration, include boric acid, sodium omadine, and 2-bromo-2-nitropropane-1,3-diol (BND). The latter compound is available under the tradename Bronopol from Boots Chemical Company, Ltd., London, United Kingdom.

The preferred embodiment of the present proposal employs bromcresol purple as the indicator compound in combination with 2-bromo-1-nitropropane-1,3-diol (BND) as the degradation inhibiting compound. The BND is colorless when added to milk. The addition of the bromcresol purple, however, causes a deep blue coloration of the milk at pH 6.7 when the milk is fresh. Such coloration informs those handling the milk samples that the preservative composition has been added and that the milk is unsuitable for consumption.

The preservative composition employing bromcresol purple provides the following color transition.

Color transition of bromcresol purple in milk

| pH | Color | Action |
|---|---|---|
| 6.7—6.3 | Blue to blue gray | Sample normal; proceed with test |
| 6.3—5.3 | Gray-green with traces of yellow to yellow-green | Examine sample for graininess, curd and/or whey formation; if none, test sample. |
| 5.3—4.6 | Yellow with no trace of green | Discard sample |

Using the preferred bromcresol purple formulation of the present proposal, personnel testing the milk samples can decide on the appropriate action depending on the color of the sample, as set forth above. When the sample is blue or blue-gray, the pH is such that no substantial degradation of the sample is likely to have occurred. When the color displays traces of yellow in combination with green, the pH is in the range where sweet-curd degradation or acid proteolysis may have occurred, depending on the microorganism involved. It is necessary to examine the sample for signs of protein precipitation, i.e., graininess, curd formation, and whey separation. If any of these are apparent, the sample should be discarded. If not, the sample may be tested. Finally, if the sample is yellow with no traces of green, the pH is such that lactic acid precipitation will almost certainly have occurred and the sample should be discarded.

The indicator compound and degradation inhibiting compound of the present teaching will normally be combined with an inert solid or liquid carrier. Conveniently, they will be combined with a solid carrier and formed into tablets of an appropriate size for treating the contemplated milk samples. The relative and absolute portions of the degradation inhibiting compound, indicator compound and vehicle will depend on the size of the milk sample to be treated as well as on the effective concentrations of the active ingredients and the desired tablet size. Bromcresol purple should be added to the tablet in an amount sufficient to provide a concentration of at least 0.01 mg/ml when added to the milk sample, usually in the range from 0.015 to 0.05 mg/ml. The effective ranges for the various preservative compounds are well known in the art.

The inert carrier will typically be a soluble salt, such as sodium chloride or potassium chloride, selected based on availability and low cost. Additional components may be added to the tablet, including anti-caking agents and lubricants which facilitate tablet formation in a machine and dispersing agents which enhance the solution of the tablets in milk. Exemplary anticaking agents, lubricants and dispersing agents are set forth below.

An exemplary tablet formation intended for treatment of 50 ml milk samples is as follows:

4

Exemplary tablet formulation[1]

| Component | Effective[6] range | Preferred[6] formula |
|---|---|---|
| Bromcresol purple | 0.50—1.0% | 0.75% |
| BND | 5.00—10% | 10.00% |
| Vehicle[2] | 0.00—95% | 47.25% |
| Anticaking agent[3] | 0.25—2.0% | 1.00% |
| Lubricant[4] | 0.25—2.0% | 1.00% |
| Dispersing agent[5] | 0.25—50% | 40.00% |

[1] Based on treatment of 50 ml milk sample.
[2] Usually KCl and/or NaCl.
[3] Silica, silicon dioxide, and combinations thereof.
[4] Stearic acid, lauric acid, oleic acid, boric acid, and combinations thereof.
[5] Glucono-δ-lactone.
[6] Percentage based on 120 mg tablet.

The exemplary tablet can be produced as follows. The crystalline components, e.g., BND, KCl, NaCl, and glucono-δ-lactone, are combined with one-half the boric acid and passed through a 20 mesh (Tyler standard) screen i.e. having an aperture size of 0.84 mm. The powder components, e.g. silicon dioxide, and stearic acid, are combined with the remaining boric acid passed through a 40 mesh Tyler standard screen, i.e. having an aperture size of 0.37 mm. The bromcresol purple is ground, typically using a mortar and pestal, with a small quantity of the crystalline components blend added. The remaining crystalline components, the bromcresol purple and the powder components are then combined and mixed in a tumbler mixer until the composition is uniform, usually taking from 20 to 40 minutes. The composition may then be compressed into tablets having a desired size.

The following experiments are offered by way of example and not by way of limitation.

Experimental

Samples (50 ml) of fresh raw milk and fresh homogenized milk having bromcresol purple added (18 mg/l) were inoculated with a fresh milk culture of *Streptococcus cremoris* (4% by volume). The samples were allowed to incubate at 31°C (88°F) for various periods of time, after which they were quickly immersed in an ice water bath. Consistency, color, pH, percent titratable acidity (%TA), and cell density (colony forming units/ml) were determined for each sample. The results are set forth in Table 1.

TABLE 1

| Sample | Measurement | Incubation period (hours) | | | | | | |
|---|---|---|---|---|---|---|---|---|
| | | 0 | 1.5 | 2.0 | 2.5 | 3.0 | 3.5 | 4.0 |
| Raw milk | pH | 6.45 | 6.30 | 6.10 | 5.99 | 5.79 | 5.65 | 5.08 |
| | %TA | 0.19 | 0.23 | 0.26 | 0.30 | 0.34 | 0.38 | 0.48 |
| | cfu/ml* | 0.66 | 1.50 | 2.10 | 3.50 | 5.70 | 8.20 | 5.60 |
| | color | blue | gray-green | yellow-green | yellow-green | light yellow green | light yellow green | yellow |
| | consistency | liquid | liquid | liquid | liquid | liquid | liquid | ppt. |
| Homo. milk | pH | 6.45 | 6.34 | 6.12 | 5.98 | 5.77 | 5.27 | 4.85 |
| | %TA | 0.19 | 0.22 | 0.26 | 0.30 | 0.35 | 0.46 | 0.54 |
| | cfu/ml* | 0.76 | 1.30 | 2.40 | 3.30 | 6.30 | 7.80 | 7.50 |
| | color | blue | gray-green | yellow-green | yellow-green | light yellow-green | yellow | bright yellow |
| | consistency | liquid | liquid | liquid | liquid | liquid | ppt. | ppt. |

*($\times 10^8$)

Samples (50 ml) of fresh raw milk and fresh homogenized milk having bromcresol purple added (18 mg/l) were treated with lactic acid to simulate the acid development caused by bacterial growth. Consistency, color, pH and %TA were determined for each sample. The results are set forth in Table 2.

TABLE 2

| Sample | Measurement | Lactic acid (10%, ml) | | | | | | |
|--------|-------------|-----|------|------|------|------|------|------|
| | | 0 | 0.5 | 1.0 | 2.0 | 3.0 | 4.0 | 5.0 |
| Raw milk | pH | 6.56 | 6.45 | 6.34 | 6.09 | 5.82 | 5.59 | 5.27 |
| | %TA | 0.16 | 0.19 | 0.22 | 0.28 | 0.34 | 0.40 | 0.46 |
| | color | blue | blue | gray-green | yellow-green | yellow-green | yellow-green | light yellow |
| | consistency | liquid | liquid | liquid | liquid | liquid | liquid | ppt. |
| Homo. milk | pH | 6.68 | 6.49 | 6.34 | 6.06 | 5.77 | 5.54 | 5.11 |
| | %TA | 0.15 | 0.18 | 0.21 | 0.27 | 0.34 | 0.40 | 0.47 |
| | color | blue | blue | gray-green | yellow-green | yellow-green | yellow-green | yellow |
| | consistency | liquid | liquid | liquid | liquid | liquid | liquid | ppt. |

These results indicate that the color indicator used in the present invention provides a useful indication of the incipient bacterial degradation of milk. Such indication allows personnel testing the milk to screen individual samples and to eliminate unsuitable samples prior to testing.

**Claims**

1. A preservative composition for addition to a milk sample while it is fresh prior to sending it to a laboratory for compositional testing, the preservative composition comprising a compound for inhibiting bacterial degradation of the milk, characterised in that an indicator compound or a combination of indicator compounds compatible with said degradation inhibiting compound is incorporated in the preservative composition for sample screening prior to compositional testing, wherein said indicator compound, when added to the milk, changes color over the pH range from 6.6 to 4.6, whereby bacterial degradation results in an observable change of color in the milk.

2. A preservative composition as in claim 1, characterised in that the indicator compound is selected from bromcresol purple, chlorphenol red, nitrazine yellow, and bromothymol blue.

3. A preservative composition as in claim 1, characterised in that the composition is formulated as a tablet having a sufficient amount of bromcresol purple as indicator compound to yield a concentration of at least 0.01 mg/l when dissolved in a sample of milk having a preselected volume.

4. A preservative composition in accordance with claim 1, characterised by 5 to 20 weight percent of 2-bromo-1-nitropropane-1,3-diol forming said degradation inhibiting compound; 0 to 95 weight percent of a water-soluble vehicle; and 0.5 to 1.0 weight percent of bromcresol purple forming said indicator compound; wherein said preservative composition is in the form of a tablet having a weight sufficient to treat a preselected milk sample size.

5. A preservative composition in accordance with claim 1, characterised in that the tablet also includes from 0.25% to 2% by weight of an anticaking agent, from 0.25 to 2% by weight of a lubricant and from 0.25 to 50% by weight of a dispersing agent.

6. A method for screening milk samples in a laboratory for bacterial degradation prior to compositional testing, said method employing a preservative composition comprising a degradation inhibiting compound and in an indicator compound or a combination of indicator compounds compatible therewith which changes color in aqueous solution over the pH range from 4.6 to 6.6, said method comprising: adding a preselected amount of the preservative composition to the milk samples while said samples are substantially fresh; observing the color of each milk sample immediately prior to compositional testing; and comparing the observed color to color standards which are indicative of the degree of bacterial degradation.

7. A method as in claim 6, wherein the indicator compound is bromcresol purple.

**Patentansprüche**

1. Konservierungsmittel zum Zusetzen zu einer im frischen Zustand befindlichen Milchprobe vor dem Versand derselben zu einem Laboratorium zur Untersuchung der Zusammensetzung, wobei das Konservierungsmittel eine Verbindung enthält zur Verhinderung bakteriellen Abbaus der Milch, dadurch gekennzeichnet, daß eine zu der Verbindung zum Verhindern des Abbaus kompatible Indikatorverbindung oder eine Kombination solcher Verbindungen in dem Konservierungsmittel aufgenommen ist zur Sichtprüfung der Proben vor der Untersuchung der Zusammensetzung, wobei die der Milch zugesetzte Indikatorverbindung in dem pH-Bereich von 6,6 bis 4,6 ihre Farbe wechselt, wodurch bei baktierellem Abbau eine beobachtbare Farbänderung der Milch erfolgt.

2. Konservierungsmittel nach Anspruch 1, dadurch gekennzeichnet, daß die Indikatorverbindung ausgewählt ist aus Bromkresolpurpur, Chlorphenolrot, Nitrazingelb und Bromthymolblau.

3. Konservierungsmittel nach Anspruch 1, dadurch gekennzeichnet, daß das Mittel als eine Tablette mit einer ausreichenden Menge Bromkresolpurpur als Indikatorverbindung vorbereitet ist, um bei Auflösen in einer Milchprobe mit einem vorgewählten Volumen eine Konzentration von mindestens 0,01 mg/l zu ergeben.

4. Konservierungsmittel nach Anspruch 1, gekennzeichnet durch 5 bis 20 Gewichtsprozent 2-Brom-1-Nitropropan-1,3-diol als die abbauverhindernde Verbindung; 0 bis 95 Gewichtsprozent eines wasserlöslichen Trägers und 0,5 bis 1,0 Gewichtsprozent Bromkresolpurpur als Indikatorverbindung, wobei das Konservierungsmittel in Form einer Tablette mit einem ausreichenden Gewicht zur Behandlung einer vorgewählten Milchprobengröße vorliegt.

5. Konservierungsmittel nach Anspruch 1, dadurch gekennzeichnet, daß die Tablette auch von 0,25 bis 2 Gewichtsprozent Antikoagulierungsmittel, von 0,25 bis 2 Gewichtsprozent Schmiermittel und von 0,25 bis 50 Gewichtsprozent Dispersionsmittel enthält.

6. Verfahren zur Sichtprüfung von Milchproben auf backteriellen Abbau vor einer Zusammensetzungs-Prüfung in einem Laboratorium, wobei bei dem Verfahren ein Konservierungsmittel benutzt wird, das eine Abbau-Verhinderungsverbindung und eine damit kompatible Indikatorverbindung oder eine Kombination solcher Indikatorverbindungen enthält, welche(s) in wässriger Lösung bei einem pH-Bereich von 4,6 bis 6,6 seine Farbe wechselt bzw. wechseln, wobei bei dem Verfahren eine vorgewählte Menge des Konservierungsmittels zu den Milchproben hinzugefügt wird, während die Milchproben im wesentlichen frisch sind, die Farbe jeder Milchprobe unmittelbar vor der Zusammensetzungsuntersuchung beobachtet und die beobachtet Farbe mit Farbnormalen verglichen wird, die für das Ausmaß des backteriellen Abbaus bezeichnend sind.

7. Verfahren nach Anspruch 6, bei dem die Indikatorverbindung Bromkresolpurpur ist.

## Revendications

1. Composition d'agent de conservation destinée à être ajoutée à un échantillon de lait à l'état frais avant d'envoyer ce dernier à un laboratoire afin d'en tester la composition, la composition d'agent de conservation comprenant un composé destiné à inhiber la dégradation bactérienne du lait, caractérisée en ce qu'un composé servant d'indicateur ou bien un mélange de composés servant d'indicateurs, compatible avec ledit composé inhibiteur de dégradation, est incorporé à la composition d'agent de conservation destinée à l'examen de l'échantillon avant d'en tester la composition, ledit composé servant d'indicateur, lorsqu'il est ajouté au lait, changeant de couleur dans l'intervalle de pH allant de 6,6 à 4,6, une dégradation bactérienne ayant pour résultat un changement de couleur observable du lait.

2. Composition d'agent de conservation suivant la revendication 1, caractérisée en ce que le composé servant d'indicateur est choisi entre le pourpre de bromocrésol, le rouge de chlorophénol, le jaune de nitrazine et le bleu de bromothymol.

3. Composition d'agent de conservation suivant la revendication 1, caractérisée en ce qu'elle est formulée sous forme d'un comprimé renfermant une quantité suffisante de pourpre de bromocrésol servant d'indicateur pour parvenir à une concentration d'au moins 0,01 mg/l lors de sa dissolution dans un échantillon de lait de volume prédéterminé.

4. Composition d'agent de conservation suivant la revendication 1, caractérisée en ce qu'elle renferme 5 à 20% en poids de 2-bromo-1-nitropropane-1,3-diol constituant le composé inhibiteur de dégradation, 0 à 95% en poids d'un véhicule hydrosoluble et 0,5 à 1,0% en poids de pourpre de bromocrésol servant d'indicateur, ladite composition d'agent de conservation étant sous forme d'un comprimé ayant un poids suffisant pour le traitement d'un volume prédéterminé d'échantillon de lait.

5. Composition d'agent de conservation suivant la revendication 1, caractérisée en ce que le comprimé renferme également 0,25% à 2% en poids d'un agent empêchant la coagulation, 0,25 à 2% en poids d'un lubrifant et 0,25 à 50% en poids d'un agent de dispersion.

6. Méthode d'examen d'échantillons de lait en laboratoire afin d'en déterminer la dégradation bactérienne avant d'en tester la composition, ladite méthode utilisant une composition d'agent de conservation comprenant un composé inhibiteur de dégradation et un composé servant d'indicateur ou un mélange de composés servant d'indicateurs, compatible avec ce composé, qui provoque un changement de coloration en solution aqueuse dans l'intervalle de pH de 4,6 à 6,6, ladite méthode consistant: à ajouter une quantité prédéterminée de la composition d'agent de conservation aux échantillons de lait lorsque lesdits échantillons sont à l'état pratiquement frais; à observer la couleur de chaque échantillon de lait immédiatement avant d'en tester la composition; et à comparer la couleur observée à des couleurs de référence, qui donnent une indication du degré de dégradation bactérienne.

7. Méthode suivant la revendication 6, dans laquelle le composé servant d'indicateur est le pourpre de bromocrésol.